# EUROPÄISCHE PATENTSCHRIFT

(11) **EP 1 641 504 B1**
(45) Veröffentlichungstag und Bekanntmachung des Hinweises auf die Patenterteilung: **14.02.2007**
(21) Anmeldenummer: 04741875.1
(22) Anmeldetag: 23.06.2004
(51) Int. Cl.: A61M 1/02

(54) **VORRICHTUNG ZUR TRENNUNG VON KOMPONENTEN EINES FLUIDS**
DEVICE FOR SEPARATING THE CONSTITUENTS OF A FLUID
DISPOSITIF POUR SEPARER DES CONSTITUANTS D'UN FLUIDE

(30) Priorität: 03.07.2003 DE 10330046; 21.10.2003 DE 20316115 U
(43) Veröffentlichungstag der Anmeldung: 05.04.2006
(73) Patentinhaber: Lmb Lab med Blutbank Technologie GmbH, 85445 Schwaig (DE); Maco Pharma International GmbH, 63225 Langen (DE)
(72) Erfinder: JENTSCH, Klaus, 85445 Oberding (DE); NOBIS, Hans-Peter, 45899 Gelsenkirchen (DE); JESSEN, Erik, S-45033 Grund Sund (SE)
(74) Vertreter: Winter, Brandl, Fürniss, Hübner Röss, Kaiser, Polte Partnerschaft Patent- und Rechtsanwaltskanzlei
(86) Internationale Anmeldenummer: PCT/EP2004/051215
(87) Internationale Veröffentlichungsnummer: WO 2005/002644

(56) Entgegenhaltungen:
- WO-A-01/24848
- DE-A- 3 815 643
- US-A- 4 663 032
- US-A- 5 472 621
- US-A1- 2002 042 336
- US-B1- 6 322 709

## Beschreibung

Die Erfindung betrifft ein Separationsverfahren zum Trennen von einem schichtweise unterteilten Fluid, insbesondere zentrifugiertem Vollblut, in seine komponenten.

Blutkonserven werden dem Empfänger einer Blutspende nicht mehr so, wie sie entnommen wurden, verabreicht. Seit geraumer Zeit hat sich bei Transfusionen die sogenannte Blutkomponententherapie durchgesetzt. Das heißt, der Patient erhält heute genau die Komponente des Vollblutes, die er benötigt.

So bekommt der Patient z.B. für die Aufrechterhaltung der Sauerstoffversorgung nach Blutverlusten die roten Blutkörperchen, bzw. Erythrozyten, bei Gerinnungsstörungen das Blutplasma und bei Gerinnungsstörungen, die auf einen zu geringen Blutplättchengehalt im Blut des Patienten zurückzuführen sind, die Blutplättchen, bzw. Thrombozyten, verabreicht.

Die Gewinnung der Blutkomponenten Erythrozyten, Blutplasma, Thrombozyten und des Leukozytenfilms bzw. Buffy Coats erfolgt in zwei unterschiedlichen Blutbeutel-Schlauch-Systemen (Top-Top-Systeme und Top-Bottom-Sys□teme), die sich bezüglich dieser Erfindung allerdings lediglich dahingehend unterschei□den, dass in dem Top-Bottom-System die parallele Gewinnung von Erythrozyten und Blutplasma möglich ist. Darüber hinaus unterscheidet man bei den Blutbeutel-Schlauch-Systemen noch nach der Anzahl der miteinander verbundenen Blutbeutel.

Die beiden verwendeten Blutbeutel-Schlauch-Systeme, die jeweils ein geschlossenes System ausbilden, enthalten von Anfang der Trennung an alle für die Gewinnung der Komponenten notwendigen Beutel, Schläuche sowie eine Nährstofflösung, wie z.B. SAG Manitol, so dass die sterile Gewinnung der Komponenten sichergestellt werden kann.

In einem Beutel des Blutbeutel-Schlauch-Systems befindet sich zentrifugiertes Vollblut. Durch das Zentrifugieren trennt sich das Blut in diesem Beutel in voneinander getrennte Schichten. Oben im Beutel befindet sich das leicht bernsteinfarbene Blutplasma, in der Mitte die Thrombozyten und der Leukozytenfilm (Buffy Coat) und unten die dunkelroten Erythrozyten.

Über ein Abpressgerät werden nun die schichtweise getrennten Komponenten aus dem ersten Beutel in weitere Beutel des Beutel-Schlauch-Systems gepresst.

Ein solches Abpressgerät ist aus der Patentschrift DE 3815643 C2 bekannt. Das Abpressgerät besteht aus einem Gehäuse mit einer daran befestigten, schwenkbar gelagerten Abpressplatte. Der Blutbeutel mit dem zentrifugierten Vollblut wird zwischen Gehäuse und Abpressplatte gelegt. Durch das Drücken der Abpressblatte in Richtung des Gehäuses wird der dazwischen befindliche Blutbeutel zusammengedrückt und die erste Komponente des Vollbluts aus einer Öffnung, am Blutbeutel oben, in einen weiteren Blutbeutel gefördert.

Das heißt im hier dargelegten Stand der Technik werden über eine Abpressplatte und die oben erläuterten Blutbeutel-Schlauch-Systeme die genannten Blutkomponenten in die einzelnen, dafür vorgesehenen Blutbeutel gefördert.

Aus dem Stand der Technik ergeben sich allerdings Nachteile, die die Qualität und die Weiterverarbeitung des gewonnenen Blutprodukts beeinträchtigt. Nach dem Abpressen einer Komponente des Vollblutes in einen anderen Blutbeutel ist eine erhöhte Kontamination mit Bakterien feststellbar. Daher ist es medizinisch erforderlich dieser Kontamination entgegenzuwirken. Darüber hinaus kommt es beim Einfrieren zu Brüchen der Blutbeutel mit Blutplasma.

Das Dokument US-B1-6322709 offenbart ein Separationsverfahren zum Trennen von einem schichtweise unterteilten Fluid, insbesondere zentrifugiertem Vollblut, in seine Komponenten, mit den Schritten: Aufnehmen eines ersten Behältnisses mit dem zu trennenden, schichtweise unterteilten Fluid in einer ersten Aufnahme, Aufnehmen eines zweiten Behältnisses, in das eine erste abgetrennte Komponente geleitet werden soll, in einer zweiten Aufnahme, Fördern dieser Komponente des Fluids aus dem ersten Behältnis zu dem mit dem ersten Behältnis in Verbindung stehenden zweiten Behältnis mittels einer ersten Fördereinrichtung, Fördern eines Fluids, nach der Trennung der Komponenten, über eine Fördereinrichtung und durch eine dritte Absperreinrichtung aus einem dritten Behältnis in das erste Behältnis, wobei eine dritte Aufnahme für das dritte Behältnis vorgesehen ist und wobei das dritte Behältnis mit dem ersten Behältnis und dem zweiten Behältnis in Verbindung steht. Dieses Dokument offenbart weiterhin den Schritt des Entfernens eines gasförmigen Mediums und dessen Beförderung in ein anderes Behältnis.

Es ist demnach Aufgabe der vorliegenden Erfindung, ein Verfahren zum Trennen von einem schichtweise unterteilten Fluid, insbesondere zentrifugiertem Vollblut bereitzustellen welches ermöglicht, die Kontamination mit Bakterien in den Behältnissen zu verringern und die Widerstandsfähigkeit beim Einfrieren zu erhöhen.

Diese Aufgabe wird durch den unabhängigen Anspruch 1 der vorliegenden Erfindung gelöst.

Vorteilhafte Ausgestaltungen der vorliegenden Erfindung sind Gegenstand der abhängigen Ansprüche.

Gemäß einer weiteren Ausgestaltung der vorliegenden Erfindung enthält die Separationsvorrichtung zur Ausführung des Separationsverfahrens eine Sensoreinrichtung, die feststellt, ob die geförderte Luft bzw. das gasförmige Medium vollständig aus dem zweiten Behältnis gefördert wurde. Vorteilhafterweise befindet sich diese Sensoreinrichtung direkt an oder in einer Absperrvorrichtung.

Zur vollautomatischen Trennung des in Schichten unterteilten Fluids ist es notwendig, dass die Separationsvorrichtung erkennen kann, ob die Luft vollständig aus dem zu entlüftenden Behältnis ausgetreten ist.

Gemäß einer weiteren Ausgestaltung der vorliegenden Erfindung weist die Separationsvorrichtung zur Ausführung des Separationsverfahrens eine Waage zum wiegen des zu trennenden Fluids und/oder mindestens eine Waage zum wiegen der einzelnen Komponenten auf.

Entsprechend dieser Ausgestaltung ist es somit möglich alle gewonnenen Komponenten zu wiegen und somit für die weiter Verarbeitung und den Trennungsvorgang der Komponenten selbst wertvolle Prozessdaten zu erlangen.

Gemäß einer anderen Ausgestaltung der Erfindung wird die Separationsvorrichtung zur Ausführung des Separationsverfahrens mit mindestens einer zweiten Sensoreinrichtung ausgestattet, die überwacht, ob die einzelnen Komponenten des zu trennenden Fluids jeweils vollständig aus dem ersten Behältnis gefördert wurden, bzw. feststellt, welche Position eine Komponente in dem zu trennenden Fluid einnimmt.

Ebenso notwendig für eine vollautomatische Trennung eines in Schichten unterteilten Fluids ist eine Sensoreinrichtung zum Überprüfen, ob die jeweilige Komponente aus dem ersten Behältnis mit dem zu trennenden Fluid vollständig ausgetreten ist. Ferner kann diese Sensoreinrichtung verwendet werden, um das Verhältnis der einzelnen Komponenten in dem ersten Behältnis zu ermitteln. In Verbindung mit den Waagen zum Ermitteln des Gewichts der Komponenten des Fluids sowie der Waage zum Ermitteln des Gewichts des schichtweise unterteilten Fluids, kann so eine Voraussage über die zu erwartenden Mengen der Komponenten getroffen werden. Am Beispiel der Blutkomponentengewinnung erklärt bedeutet das, dass ein Hämatokritwert mittels der Sensoreinrichtung fortwährend bestimmt wird und anhand des Ausgangsgewichts und der Gewichte der Komponenten bestimmt werden kann welche Menge einer Blutkomponente zu erwarten ist und wann mit Beginn der Förderung der nächsten Blutkomponente gerechnet werden kann.

Die vorliegende Erfindung gewährleistet, dass keine Luft aus dem in der Separationsvorrichtung befindlichen System in die Umwelt entweicht. Dadurch kann sichergestellt werden, dass z.B. bei einem Blutbeutel-Schlauch-System die Blutkomponenten völlig steril gehalten werden können.

Gemäß einer weiteren Ausgestaltung der Erfindung werden weitere Aufnahmen 4 und 5 bereitgestellt, um weitere Komponenten des zu trennenden Fluids in daran befestigten Behältnissen aufzunehmen. Es können somit insbesondere für die Verwendung zur Trennung von Vollblut alle üblichen Blutbeutel-Schlauch-Systeme mit bis zu fünf Blutbeuteln verwendet werden.

Gemäß einer anderen Ausgestaltung der Erfindung ist die zweite Fördereinrichtung und die dritte Fördereinrichtung als eine kombinierte Fördereinrichtung ausgebildet. Das kann z.B. durch eine bidirektional wirkende Abpressplatte geschehen, die in eine erste Richtung das dritte Behältnis, z.B. den Blutbeutel mit Nährstofflösung zusammenpressen kann, und in eine zweite Richtung das zweite Behältnis, z.B. den Blutbeutel mit Blutplasma zusammendrücken kann.

Diese Ausgestaltung der Erfindung ermöglicht es den Kompaktheitsgrad der Sepa-rationsvorrichtung zu erhöhen und dadurch die Abmessungen der Vorrichtung zu verkleinern.

Gemäß einer Ausgestaltung der Erfindung ist eine Verschlusseinrichtung vorgesehen, um nach Beendigung des Trennungsvorgangs die Verbindung zwischen den Behältnissen, d.h. vorzugsweise Schlauchverbindungen aus Kunststoff, dauerhaft zu verschließen. Vorteilhaft ist es diese Verschlusseinrichtung in oder an den Absperreinrichtungen vorzusehen. Für den Fall, dass es sich um Kunststoffschläuche handelt werden diese Verschlusseinrichtungen Schlauchschweißknöpfe genannt und haben die Aufgabe den Kunststoff mittels eines Hochfrequenzstroms zu verschweißen.

Gemäß einer anderen Ausgestaltung der Erfindung weist die Separationsvorrichtung zur Ausführung des Separationsverfahrens eine Steuereinheit auf, die den Separationsvorgang vollautomatisch durch Ansteuern von Förder-, Absperr- und Verschlusseinrichtungen auf Basis der Signaleingänge von den Waagen und/oder der Sensoreinrichtungen regelt und gewonnene Prozessdaten speichert.

Diese Ausgestaltung macht es möglich, dass das Bedienpersonal der Separationsvorrichtung nur noch die entsprechenden Behältnisse, z.B. das Blutbeutel-Schlauch-System, einlegen und aus der Separationsvorrichtung entnehmen muss. Somit kann Arbeitszeit eingespart werden und es können Bedienfehler vermieden werden.

Gemäß einer weiteren Ausgestaltung der Erfindung werden Prozessdaten, die während des Trennungsvorgangs ermittelt werden, von der Steuereinheit an eine Datenverarbeitungseinrichtung übertragen.

Die Datenübertragung zwischen Steuereinheit und Datenverarbeitungseinrichtung kann z.B. über Wireless LAN erfolgen und ermöglicht es die Daten aus dem Trennungsprozess weiter zu verarbeiten und zu verwenden. Das Übertragen von während der Trennung gewonnenen Daten von Hand entfällt.

Gemäß einer anderen Ausgestaltung der Erfindung enthält die Separationsvorrichtung zur Ausführung des Separationsverfahrens Schrittmotoren, die abhängig von den Eingängen von der ersten und/oder der zweiten Sensoreinrichtung so angesteuert werden, dass die Separationsvorrichtung den für die Trennung benötigten Druck in den Behältnissen aufbaut.

Gemäß einer Ausgestaltung der Erfindung weist die Separationsvorrichtung zur Ausführung des Separationsverfahrens ferner ein Weichfiltergehäuse für einen Weichfilter auf, durch den die zu trennende Komponente fließt. Die Filterwirkung des Weichfilters lässt nach, sobald der Weichfilter sich aufgrund des durch ihn fließenden Fluids zu weit aufbläht. Das Volumen, das der Weichfilter einnimmt, kann mittels des Weichfiltergehäuses begrenzt werden, die Filterwirkung kann somit konstant gehalten werden.

Gemäß einer weiteren Ausgestaltung der Erfindung weist die Separationsvorrichtung zur Ausführung des Separationsverfahrens und mindestens ein Behältnis ein sogenanntes Smartlabel System auf, wie sie vom Anmelder vorliegender Erfindung bereits in der Offenlegungsschrift DE 10059 539 A1 vorgeschlagen wurde. Bei dem Smartlabel System handelt es sich um eine Vorrichtung zum Überwachen und Verwalten von Produkten, die in oder an einem Produkt angebracht werden kann. Die Vorrichtung weist eine Sende/Empfangsvorrichtung auf, in der das Produkt betreffende Daten speicherbar sind. Die das Produkt betreffenden Daten sind mittels einer externen Schreib/Lesevorrichtung berührungslos in die Sende/Empfangsvorrichtung schreibbar und aus dieser lesbar. Die Vorrichtung weist weiterhin eine Sensorvorrichtung auf, die einen zeitlich veränderlichen Zustandsparameter des Produkts überwacht und in Abhängigkeit von einem erfassten Wert des zeitlich veränderlichen Zustandsparameters einem aktuell vorliegenden Zustand entsprechende Daten zu der Sende/Empfangsvorrichtung überträgt. Die dem aktuell vorliegenden Zustand entsprechenden Daten werden als Reaktion auf das Übertragen von der Sensorvorrichtung in die Sende/Empfangsvorrichtung geschrieben.

Beim Beispiel der Blutkomponentengewinnung können somit mittels dieser Technologie Daten direkt an dem Blutbeutel mit einer Blutkomponente gespeichert werden, die beispielsweise eine Herstellungsnummer, das Datum, die Uhrzeit oder die Menge des hergestellten Produkts betreffen.

Die vorliegende Erfindung wird nachstehend anhand von Ausführungsbeispielen unter Bezugnahme auf die beiliegende Zeichnung detailliert erläutert.

Es zeigt:

Fig. 1 eine erste Ausführungsform einer Separationsvorrichtung mit einem Top-Top-System bestehend aus vier Blutbeuteln.

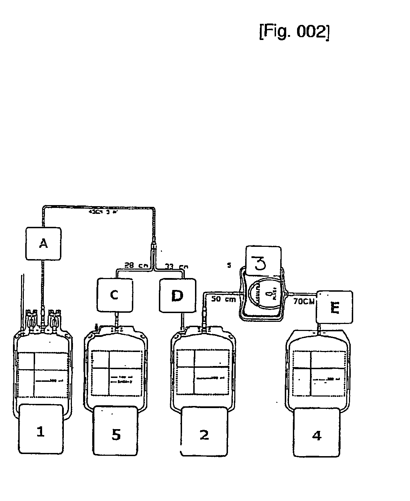

Fig. 2 zeigt ein Blutbeutelsystem, ausgeführt als Top-Top-System mit vier Blutbeuteln.

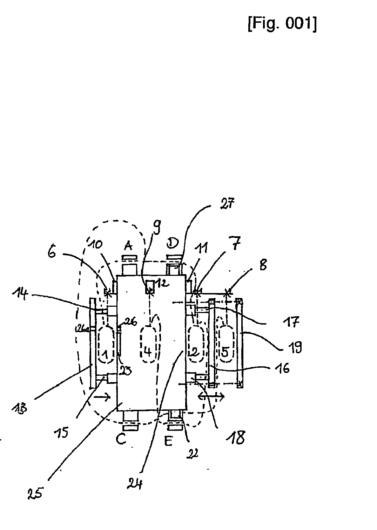

Fig. 1 zeigt eine Ausführungsform der vorliegenden Erfindung, die zur Bearbeitung eines Top-Top-Systems bestehend aus vier Blutbeuteln geeignet ist.

Eine erste Aufhängung 6 ist an einer Waage 10 am Gehäuse 25 der Separationsvorrichtung befestigt. Die Aufhängung 6 dient dazu, einen ersten Blutbeutel 1 eines geschlossenen und sterilen Blutbeutelsystems aufzuhängen und zu wiegen. Der erste Blutbeutel 1 enthält zentrifugiertes Vollblut. Der Blutbeutel 1 hängt zwischen einer Abpressplatte 13 und dem Gehäuse 25, das als Anpresswand dient. Eine erste zu dem Blutbeutel-Schlauch-System gehörende Schlauchverbindung zwischen dem Blutbeutel 1 und dem Blutbeutel 2, der das Blutplasma des zentrifugierten Vollbluts aufnimmt, wird durch die Klemm- und Verschlussköpfe A und D geführt. Zum Abpressen des Blutplasmas aus dem ersten Blutbeutel 1 werden nun die Pneumatikzylinder 14 und 15 bzw. Schrittmotoren (nicht dargestellt) betätigt, so dass die Abpressplatte 13 in Richtung des Gehäuses 25 bewegt wird, und in dem zwischen der Abpressplatte 13 und dem Gehäuse 25 eingeklemmten Beutel Druck aufgebaut wird. Das Blutplasma wird durch die Schlauchleitung über den geöffneten Klemm- und Verschlusskopf A und den geöffneten Klemm- und Verschlusskopf D in den Plasmabeutel 2 geleitet. Bei diesem Vorgang ist der Klemm- und Verschlusskopf C geschlossen, durch den eine Schlauchleitung zu einem vierten Behältnis geführt wird, so dass Blutplasma nur in den Plasmabeutel 2 gelangen kann.

Die Klemm- und Verschlussköpfe sind, um eine Klemmwirkung zu erreichen, mit einem Elektromagneten und einer Metallfeder ausgestattet. Liegt ein Signal an dem Elektromagneten an, so wird die Schlauchverbindung geklemmt und kein Durchfluss ist möglich. Liegt kein Signal an dem Elektromagneten an, so entlastet die Metallfeder die Schlauchverbindung in dem Klemm- und Verschlusskopf.

Bei dem Abpressen des Blutplasmas wird über Fotozellen 26 überwacht, ob das Blutplasma vollständig aus dem Blutbeutel 1 gefördert wurde. Die Fotozelle 26 nimmt dazu Licht von einer Lichtquelle 26a auf, das durch das anfänglich in dem Blutbeutel befindliche Blutplasma hindurchdringt. So lange das Licht durch das Blutplasma hindurchdringt, wird ein Signal von der Fotozelle 26 ausgegeben. Sobald allerdings die nächste Schicht in dem zentrifugierten Blutbeutel erreicht ist, d.h. sobald der Buffy Coat sich zwischen Lichtquelle 26a und Fotozelle 26 schiebt, wird kein Signal mehr von der Fotozelle 26 ausgegeben, und eine Steuereinheit (nicht dargestellt) erfasst, dass das Blutplasma vollständig aus dem Blutbeutel gepresst wurde. Das Abpressen des Blutplasmas wird beendet. Bei der Steuereinheit kann es sich beispielsweise um einen Singleboard PC mit PC 104 Bus als Verbindung zu den Sensoren und Aktoren handeln.

Eine vor der Trennung vorgenommene Einstellung der Menge des Buffy Coats wird nun zusätzlich zu dem Blutplasma von dem Blutbeutel 1 in den Plasmabeutel 2 gefördert. Die Förderdauer bestimmt sich dabei durch die von der Waage 10 festgestellte Gewichtsreduktion in dem Blutbeutel 1. Nach Abpressen des Buffy Coats in den Blutplasmabeutel 2 wird der Klemm- und Verschlusskopf D geschlossen und der Klemm- und Verschlusskopf C geöffnet. Daraufhin wird die Abpressplatte 16 der kombinierten Fördereinrichtung über die Pneumatikzylinder 17 und 18 in Richtung der Anpressplatte 19 gedrückt. Der Blutbeutel mit Nährlösung, wie z.B. SAG-Manitol, der sich an einer Aufhängung 8 befindet, wird zusammengedrückt bis der Inhalt des Beutels 5 vollständig in den Beutel 1 durch die Klemm- und Verschlussköpfe C und A in den Beutel 1 gefördert wurde. Anschließend wird der Klemm- und Verschlusskopf C geschlossen, und somit die Schlauchverbindung zwischen dem Beutel 5 und dem Blutbeutel 1 abgeklemmt.

Anschließend bewegen sich die Zylinder 17 und 18 in die entgegengesetzte Richtung, um die Abpressplatte 16 in Richtung des Gehäuses 25 zu bewegen, das auf Höhe des Plasmabeutels 2 als Anpressplatte 24 dient. Der Druck in dem Plasmabeutel 2 wird erhöht bis die vorbestimmte Menge an Buffy Coat, die über die Waage 11 festgestellt werden kann, in den Blutbeutel für das Buffy Coat 4 über den Klemm- und Verschlusskopf E gelangt ist. Anschließend wird der Klemm- und Verschlusskopf E wieder geschlossen und der Klemm- und Verschlusskopf D geöffnet, sowie der Klemm- und Verschlusskopf A geschlossen, um die in dem Plasmabeutel 2 auf der Oberseite befindliche Luft nunmehr in den Nährstoffbeutel 5 zu leiten. Die Abpressplatte 16 komprimiert dabei weiterhin den Blutbeutel 2.

Um festzustellen, ob die Luft vollständig aus dem Plasmabeutel 2 gedrückt wurde, wird eine Fotozelle 27 in dem Klemm- und Verschlusskopf D angeordnet, die z.B. auf Basis von ausgesendetem und reflektiertem Licht feststellen kann, wenn bereits Blutplasma durch die Schlauchverbindung in dem Klemm- und Verschlusskopf D angelangt ist.

Wird bereits Blutplasma in den Klemm- und Verschlusskopf D geleitet, klemmt der Klemm- und Verschlusskopf D die in ihr befindliche Schlauchleitung ab und der Luftabpressvorgang wird beendet.

Es befinden sich nunmehr in dem Blutbeutel 1 die Erythrozyten mit der Nährstofflösung SAG-Manitol, in dem Plasmabeutel 2 das Blutplasma ohne die bakterienhaltige Luft, die sich nun in dem nicht mehr benötigten Nährstoffbeutel 5 befindet, und in dem Blutbeutel 4 befindet sich der Buffy Coat.

Die in den Klemm- und Verschlussköpfen A, C, D, E befindlichen Schlauchteile werden eingeklemmt und über ein Hochfrequenzstromsignal eines Hochfrequenzgenerators (nicht dargestellt) in den Klemm- und Verschlussköpfen A, C, D, E verschweißt. Ferner wird die Schlauchverbindung an der entsprechenden Stelle bereits vorperforiert, so dass die Schlauchverbindung an diesen Stellen nach Entnahme aus den Klemm- und Verschlussköpfen A, C, D, E durch einfaches Zusammenlegen der Schlauchverbindung getrennt werden kann. Die Schlauchverbindung und damit der Blutbeutel mit dem Blutprodukt ist luftdicht verschlossen bzw. verschweißt.

In einer zweiten Ausführungsform der Erfindung wird anstelle des Top-Top-Systems mit vier Beuteln ein Top-Bottom-System mit fünf Beuteln verwendet. Das Top-Bottom-System zeichnet sich dadurch aus, dass die Anschlüsse an den Blutbeuteln 1 nicht nur oben, sondern oben und unten angesiedelt sind. Dadurch wird es ermöglicht, dass das Blutplasma nach oben abgepresst wird und gleichzeitig die Erythrozyten nach unten abgepresst werden, wobei sich der Buffy Coat bzw. Leukozytenfilm in der Mitte der abgepressten Komponenten befindet.

Der Abpressvorgang aus dem Blutbeutel 1 wird dann derart gesteuert, dass je nach Lage der Buffy Coat Schicht, die durch den Buffy Coat Sensor 23 festgestellt wird, der Klemm- und Verschlusskopf A und ein Klemm- und Verschlusskopf B (nicht dargestellt) geöffnet oder geschlossen werden, so dass z.B. Blutplasma durch den Klemm- und Verschlusskopf A gefördert wird, wenn sich die Buffy Coat Schicht von der Mitte des Blutbeutels 1 aus unten befindet. In dieser Ausführungsform der Erfindung werden bis zu zwölf Doppelsensoren als Buffy Coat Sensor 23 verwendet, deren Funktionsweise der der Fotozelle 26 mit entsprechender Lichtquelle 26a entspricht, aber auch jede andere Form der Ermittlung der Position des Buffy Coats ist denkbar.

Sollte sich die Buffy Coat Schicht von der Mitte des Blutbeutels 1 aus oben befinden, so wird der Klemm- und Verschlusskopf A geschlossen und der Klemm- und Verschlusskopf B geöffnet, so dass Erythrozyten aus dem Blutbeutel 1 in einen Erythrozytenbeutel 3 (nicht dargestellt) gelangen können. Ziel ist es dabei, den Buffy Coat in der Mitte des Blutbeutels 1 zu halten. Anschließend wird ähnlich der ersten Ausführungsform die Luft aus dem Plasmabeutel gedrückt, wobei Abpressplatten und Klemm- und Verschlussköpfe entsprechend angesteuert werden.

Die Separationsvorrichtung zur Ausführung des Separationsverfahrens der vorliegenden Erfindung ist so ausgelegt, dass alle kommerziell erhältlichen Blutbeutelsysteme, ob Top-Top-System oder Top-Bottom-System, ob mit drei Beuteln oder mit vier und fünf Beuteln verwendet werden können. Außerdem können in den Blutbeutel-Schlauch-Systemen Filter, wie z.B. Plasmafilter, enthalten sein. Diese werden in dafür vorgesehene Weichfiltergehäuse eingelegt, um die Filterwirkung der Weichfilter zu verbessern. Die Klemm- und Verschlussköpfe A bis E werden, wie auch die Abpressplatten, entsprechend angesteuert, so dass das zentrifugierte Vollblut in den jeweiligen Blutbeutel-Schlauch-Systemen getrennt werden kann, sofern von dem Bedienpersonal das jeweilige Beutelsystem über eine Mensch-Maschine-Schnittstelle eingegeben wurde.

Ferner wird darauf hingewiesen, dass die Separationsvorrichtung zur Ausführung des Separationsverfahrens der vorliegenden Erfindung verschiedene Überprüfungsmechanismen besitzt, um festzustellen, ob die richtigen Blutbeutel an der richtigen Aufhängung, bzw. Waage hängen, und ob die sich die Schlauchteile in den dafür vorgesehenen Klemm- und Verschlussköpfen befinden.

Darüber hinaus können Barcodes bzw. Smartlabel, die das Spenderblut identifizieren von der Separationsvorrichtung eingelesen werden.

## Patentansprüche

1. Separationsverfahren zum Trennen von einem schichtweise unterteilten Fluid, insbesondere zentrifugiertem Vollblut, in seine Komponenten, mit den Schritten:
Aufnehmen eines ersten Behältnisses (1) mit dem zu trennenden, schichtweise unterteilten ersten Fluid in einer ersten Aufnahme (6),
Aufnehmen eines zweiten Behältnisses (2), in das eine erste abgetrennte Komponente geleitet werden soll in einer zweiten Aufnahme (7),
Fördern dieser Komponente des Fluids aus dem ersten Behältnis (1) zu dem mit dem ersten Behältnis (1) in Verbindung stehenden zweiten Behältnis (2) mittels einer ersten Fördereinrichtung (13, 14, 15),
Fördern eines zweiten Fluids, nach der Trennung der Komponenten, über eine dritte Fördereinrichtung und durch eine dritte Absperreinrichtung (C) aus einem dritten Behältnis (5) in das erste Behältnis (1), wobei eine dritte Aufnahme für das dritte Behältnis (5) vorgesehen ist und wobei das dritte Behältnis (5) mit dem ersten Behältnis (1) und dem zweiten Behältnis (2) in Verbindung steht,
Entfernen eines in dem zweiten Behältnis (2) befindlichen gasförmigen Mediums aus dem zweiten Behältnis mittels einer zweiten Fördereinrichtung (16, 17, 18), und
Fördern des gasförmigen Mediums aus dem zweiten Behältnis (2) in das dritte Behältnis (5) durch die zweite Fördereinrichtung.

2. Separationsverfahren nach Anspruch 1, das den Schritt eines Unterbrechens der Verbindung zwischen dem ersten Behältnis (1) und dem zweiten Behältnis (2) mittels einer ersten Absperreinrichtung (A) aufweist, wenn die erste Komponente in das zweite Behältnis (2) gefördert wurde.

3. Separationsverfahren nach Anspruch 1 oder 2, wobei die erste Fördereinrichtung (13, 14, 15) und die zweite Fördereinrichtung (16, 17, 18) als eine einzige Fördereinrichtung ausgebildet sind.

4. Separationsverfahren nach einem der Ansprüche 1 bis 3, das den Schritt eines luftdichten Verschließens des zweiten Behältnisses (2) nach dem vollständigen Entfernen des gasförmigen Mediums aus dem zweiten Behältnis (2) mittels einer Absperreinrichtung (D) aufweist.

5. Separationsverfahren nach einem der vorherigen Ansprüche, das den Schritt eines Feststellens, ob das gasförmige Medium vollständig aus dem zweiten Behältnis gefördert wurde, mittels einer ersten Sensoreinrichtung (22), insbesondere ausgebildet an einer Absperreinrichtung, aufweist.

6. Separationsverfahren nach einem der vorherigen Ansprüche, das den Schritt eines Überwachens, ob die Komponenten jeweils vollständig aus dem ersten Behältnis (1) gefördert wurden und/oder eines Überwachens der Position einer einzelnen Komponente und damit dem Verhältnis der einzelnen Komponenten zueinander mittels mindestens einer zweiten Sensoreinrichtung (23) aufweist.

7. Separationsverfahren nach Anspruch 1, wobei die zweite Fördereinrichtung (16. 17, 18) und die dritte Fördereinrichtung als eine kombinierte Fördereinrichtung (16, 17, 18, 19) ausgebildet sind.

8. Separationsverfahren nach einem der Ansprüche 1 bis 7, wobei es sich bei einer Fördereinrichtung um mindestens einen auf eine Abpressplatte (13, 16) einwirkenden unidirektionalen Pneumatikzylinder zum Zusammendrücken eines zwischen einer Anpresswand (19, 24) und der Abpressplatte befindlichen Behältnisses handelt, so dass Druck in dem Behältnis aufgebaut wird.

9. Separationsverfahren nach Anspruch 7, wobei es sich bei der kombinierten Fördereinrichtung (16, 17, 18) um einen auf eine Abpressplatte (16) einwirkenden bidirektionalen Pneumatikzylinder zum Zusammendrücken eines zwischen einer ersten Anpresswand (19) und einer ersten Abpressplatte (16) befindlichen Behältnisses und zum Zusammendrücken eines zwischen einer zweiten Anpresswand (24) und einer zweiten Abpressplatte (16) befindlichen Behältnisses handelt.

10. Separationsverfahren nach einem der Ansprüche 1 bis 9, wobei es sich bei den in Verbindung stehenden Behältnissen (1, 2, 4, 5) um ein über Schläuche verbundenes, geschlossenes und steriles System von Plastikbeuteln handelt.

11. Separationsverfahren nach Anspruch 10, wobei es sich bei dem System von Plastikbeuteln um ein Top-Top-System oder ein Top-Bottom-System mit bis zu fünf Plastikbeuteln handelt.

12. Separationsverfahren nach einem der vorherigen Ansprüche, wobei der Separationsvorgang durch eine Steuereinheit vollautomatisch durch Ansteuern der Förder-, Absperr- und Verschlusseinrichtungen auf Basis der Signaleingänge von den Waagen und/oder Sensoreinrichtungen geregelt und gewonnene Prozessdaten gespeichert werden.

13. Separationsverfahren nach Anspruch 12, das den Schritt eines Übertragens von Prozessdaten von der Steuereinheit zu einer Datenverarbeitungseinrichtung aufweist.

14. Separationsverfahren nach Anspruch 13, wobei die Datenübertragung zwischen der Steuereinheit und der Datenverarbeitungseinrichtung über ein drahtloses LAN erfolgt.

15. Separationsvertahren nach einem der vorherigen Ansprüche, wobei es sich bei dem zu trennenden, schichtweise unterteilten Fluid um zentrifugiertes Vollblut und bei den Komponenten um Blutkomponenten, insbesondere das Blutplasma, den Leukozytenfilm und die Erythrozyten, handelt.

16. Separationsverfahren nach Anspruch 15, das den Schritt eines Ermittelns eines Hämatokritwerts des Vollbluts mittels einer Hämatokritermittlungseinrichtung zur Überwachung des Separationsvorgangs aufweist.

17. Separationsverfahren nach einem der Ansprüche 1 und 7 bis 16, wobei es sich bei dem Fluid in dem dritten Behältnis um eine Nährlösung und insbesondere SAG Manitol handelt.

18. Separationsverfahren nach einem der vorherigen Ansprüche, wobei ein Gehäuse für einen Weichfilter vorgesehen ist.

## Claims

1. Separation method for separating into its constituents a fluid that is subdivided into layers, especially centrifuged whole blood, said method comprising the following steps:
a first container (1) holding the first fluid to be separated, that is subdivided into layers, is placed in a first receptacle (6),
a second container (2), into which a first separated constituent is to be conducted, is placed in a second receptacle (7),
this constituent of the fluid is delivered, by means of a first delivery device (13, 14, 15), from the first container (1) to the second container (2) which communicates with the first container (1),
a second fluid, after the separation of the constituents, is delivered via a third delivery device and through a third shut-off device (C) from a third container (5) into the first container (1), a third receptacle being provided for the third container (5), and the third container (5) communicating with the first container (1) and with the second container (2),
a gaseous medium located in the second container (2) is removed from said second container by means of a second delivery device (16, 17, 18), and
the gaseous medium is delivered from the second container (2) into the third container (5) by the second delivery device.

2. Separation method according to Claim 1, comprising the step of interrupting the connection between the first container (1) and the second container (2) by means of a first shut-off device (A) when the first constituent has been delivered into the second container (2).

3. Separation method according to Claim 1 or 2, in which the first delivery device (13, 14, 15) and the second delivery device (16, 17, 18) are designed as a single delivery device.

4. Separation method according to one of Claims 1 to 3, comprising the step of closing the second container (2) in an air-tight manner by means of a shut-off device (D) after the complete removal of the gaseous medium from the second container (2).

5. Separation method according to one of the preceding claims, comprising the step of determining whether the gaseous medium has been delivered completely from the second container, by means of a first sensor device (22), formed in particular on a shut-off device.

6. Separation method according to one of the preceding claims, comprising the step of monitoring whether the constituents have each been delivered completely from the first container (1) and/or of monitoring the position of an individual constituent and, therefore, the ratio of the individual constituents, by means of at least one second sensor device (23).

7. Separation method according to Claim 1, in which the second delivery device (16, 17, 18) and the third delivery device are designed as a combined delivery device (16, 17, 18, 19).

8. Separation method according to one of Claims 1 to 7, in which a delivery device comprises at least one pneumatic cylinder acting in one direction on a pressure plate (13, 16) in order to compress a container located between a contact pressure wall (19, 24) and the pressure plate, so that pressure is built up in the container.

9. Separation method according to Claim 7, in which the combined delivery device (16, 17, 18) comprises a pneumatic cylinder acting in two directions on a pressure plate (16) in order to compress a container located between a first contact pressure wall (19) and a first pressure plate (16) and in order to compress a container located between a second contact pressure wall (24) and a second pressure plate (16).

10. Separation method according to one of Claims 1 to 9, in which the communicating containers (1, 2, 4, 5) form a closed and sterile system of plastic bags connected by tubes.

11. Separation method according to Claim 10, in which the system of plastic bags is a top-top system or a top-bottom system with up to five plastic bags.

12. Separation method according to one of the preceding claims, in which the separation procedure is regulated fully automatically by a control unit which regulates the delivery, shut-off and closure devices on the basis of signal inputs from balances and/or sensor devices, and in which the process data obtained are stored.

13. Separation method according to Claim 12, comprising the step of transferring process data from the control unit to a data processor.

14. Separation method according to Claim 13, in which the data transfer between the control unit and the data processor is carried out via a wireless LAN.

15. Separation method according to one of the preceding claims, in which the fluid to be separated, that is subdivided into layers, is centrifuged whole blood, and the constituents are blood constituents, in particular the blood plasma, the buffy coat and the erythrocytes.

16. Separation method according to Claim 15, comprising the step of determining a haematocrit value of the whole blood by means of a haematocrit determination device in order to monitor the separation procedure.

17. Separation method according to one of Claims 1 and 7 to 16, in which the fluid in the third container is a nutrient solution, and in particular SAG-mannitol.

18. Separation method according to one of the preceding claims, in which a housing for a soft filter is provided.

## Revendications

1. Procédé de séparation pour séparer les composants d'un fluide subdivisé en couches, notamment du sang complet centrifugé, comprenant les étapes suivantes :
fixation dans un premier réceptacle (6) d'un premier récipient (1) avec le premier fluide subdivisé en couches à séparer,
fixation dans un deuxième réceptacle (7) d'un deuxième récipient (2) où un premier composant séparé doit être versé,
refoulement de ce composant du fluide du premier récipient (1) vers le deuxième récipient (2) raccordé au premier récipient (1), au moyen d'un premier dispositif de refoulement (13, 14, 15),
refoulement d'un deuxième fluide après séparation des composants, au moyen d'un troisième dispositif de refoulement et par un troisième dispositif d'arrêt (C), d'un troisième récipient (5) vers le premier récipient (1), un troisième réceptacle étant prévu pour le troisième récipient (5), et le troisième récipient (5) étant raccordé au premier récipient (1) et au deuxième récipient (2),
évacuation hors du deuxième récipient (2) d'un agent gazeux présent dans le deuxième récipient, au moyen d'un deuxième dispositif de refoulement (16, 17, 18), et
refoulement de l'agent gazeux du deuxième récipient (2) vers le troisième récipient (5) au moyen du deuxième dispositif de refoulement.

2. Procédé de séparation selon la revendication 1, comprenant l'étape d'interruption du raccord entre le premier récipient (1) et le deuxième récipient (2) au moyen d'un premier dispositif de fermeture (A), une fois le premier composant refoulé dans le deuxième récipient (2).

3. Procédé de séparation selon la revendication 1 ou la revendication 2, où le premier dispositif de refoulement (13, 14, 15) et le deuxième dispositif de refoulement (16, 17, 18) sont réalisés comme un dispositif de refoulement unique.

4. Procédé de séparation selon l'une des revendications 1 à 3, comprenant l'étape d'une fermeture étanche à l'air du deuxième récipient (2) après évacuation complète de l'agent gazeux hors du deuxième récipient (2), au moyen d'un dispositif de fermeture (D).

5. Procédé de séparation selon l'une des revendications précédentes, comprenant l'étape de vérification si l'agent gazeux a été complètement refoulé du deuxième récipient, au moyen d'un premier dispositif à capteur (22), réalisé en particulier sur un dispositif de fermeture.

6. Procédé de séparation selon l'une des revendications précédentes, comprenant l'étape de surveillance si chaque composant a été complètement refoulé du premier récipient (1) et/ou de surveillance de position d'un composant individuel et donc du rapport des différents composants entre eux au moyen d'au moins un deuxième dispositif à capteur (23).

7. Procédé de séparation selon la revendication 1, où le deuxième dispositif de refoulement (16, 17, 18) et le troisième dispositif de refoulement sont réalisés comme dispositif de refoulement combiné (16, 17, 18, 19).

8. Procédé de séparation selon l'une des revendications 1 à 7, où un dispositif de refoulement est au moins un vérin pneumatique unidirectionnel agissant sur une plaque de pressage (13, 16) pour la compression d'un récipient disposé entre une paroi de serrage (19, 24) et la plaque de pressage, de manière à créer une pression dans le récipient.

9. Procédé de séparation selon la revendication 7, où le dispositif de refoulement combiné (16, 17, 18) est un vérin pneumatique bidirectionnel agissant sur une plaque de pressage (16) pour la compression d'un récipient disposé entre une première paroi de serrage (19) et une première plaque de pressage (16) et pour la compression d'un récipient disposé entre une deuxième paroi de serrage (24) et une deuxième plaque de pressage (16).

10. Procédé de séparation selon l'une des revendications 1 à 9, où les récipients raccordés (1, 2, 4, 5) consistent en un système fermé et stérile de poches plastiques raccordées par des tuyaux flexibles.

11. Procédé de séparation selon la revendication 10, où le système de poches plastiques est un système haut-haut ou un système haut-bas avec jusqu'à cinq poches plastiques.

12. Procédé de séparation selon l'une des revendications précédentes, où le processus de séparation est régulé par une unité de commande de manière entièrement automatique par commande des dispositifs de refoulement, de fermeture et de verrouillage sur la base des signaux d'entrée provenant des balances et/ou de dispositifs à capteurs, et où les données de processus obtenues sont mémorisées.

13. Procédé de séparation selon la revendication 12, comprenant l'étape de transfert de données de processus de l'unité de commande vers un dispositif de traitement de données.

14. Procédé de séparation selon la revendication 13, où le transfert de données est effectué entre l'unité de commande et le dispositif de traitement de données au moyen d'un réseau local (LAN) sans fil.

15. Procédé de séparation selon l'une des revendications précédentes, où le fluide subdivisé en couches à séparer est du sang complet centrifugé, et où les composants sont des composants sanguins, en particulier le plasma sanguin, la couches de leucocytes et les érythrocytes.

16. Procédé de séparation selon la revendication 15, comprenant l'étape de détermination d'une valeur d'hématocrite du sang complet au moyen d'un dispositif de détermination du taux d'hématocrite pour la surveillance du processus de séparation.

17. Procédé de séparation selon l'une des revendications 1 et 7 à 16, où le fluide du troisième récipient est une solution nutritive, en particulier du SAG Mannitol.

18. Procédé de séparation selon l'une des revendications précédentes, où un boîtier est prévu pour un filtre mou.
